(19) Europäisches Patentamt
European Patent Office
Office européen des brevets

(11) Veröffentlichungsnummer: **0 264 752**
**B1**

(12) # EUROPÄISCHE PATENTSCHRIFT

(45) Veröffentlichungstag der Patentschrift: **29.08.90**

(51) Int. Cl.[5]: **C07C 273/18**

(21) Anmeldenummer: **87114817.7**

(22) Anmeldetag: **10.10.87**

(54) **Verfahren zur Herstellung von N,N-Diaryl-harnstoffen.**

(30) Priorität: **24.10.86 DE 3636190**

(43) Veröffentlichungstag der Anmeldung: **27.04.88 Patentblatt 88/17**

(45) Bekanntmachung des Hinweises auf die Patenterteilung: **29.08.90 Patentblatt 90/35**

(84) Benannte Vertragsstaaten: **BE CH DE ES FR GB IT LI NL SE**

(56) Entgegenhaltungen:
**EP-A- 0 066 922**
**US-A- 2 683 727**

**HOUBEN-WEYL, Band E4, 1983, Georg Thieme Verlag, Stuttgart, Seite 182, Absatz 2 in Verbindung mit Seite 353, Absatz 1**

(73) Patentinhaber: **BAYER AG, D-5090 Leverkusen 1 Bayerwerk(DE)**

(72) Erfinder: **Kissener, Wolfram, Dr., Haupstrasse 336, D-5060 Bergisch-Gladbach 2(DE)**
Erfinder: **Franke, Joachim, Dr., An der Ruthen 2, D-5000 Koeln 80(DE)**
Erfinder: **Fiege, Helmut, Dr., Walter-Flex-Strasse 23, D-5090 Leverkusen 1(DE)**
Erfinder: **Wedemeyer, Karlfried, Dr., Bilharzstrasse 7, D-5000 Koeln 80(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren zur Herstellung von N,N-Diaryl-harnstoffen durch Umsetzung von Diarylaminen mit Isocyanaten in Gegenwart von sauer reagierenden Verbindungen.

Die Herstellung von symmetrischen und unsymmetrischen Harnstoffen durch Umsetzung von Aminen mit Isocyanaten ist bereits bekannt (Houben-Weyl, Methoden der organischen Chemie, Band VI-II (1952), S. 157). Sofern es erforderlich ist, wird hierbei in Gegenwart von basischen Katalysatoren gearbeitet. Bei schwierig verlaufenden Umsetzungen, beispielsweise mit sterisch gehinderten Aminen oder solchen mit geringer Nukleophilie, wie Diarylaminen, sind auch in Gegenwart solcher basischer Katalysatoren drastische Reaktionsbedingungen erforderlich, wie extrem lange Reaktionszeiten, hohe Reaktionstemperaturen oder große Überschüsse an Isocyanat. Bei solchen verschärften Reaktionsbedingungen sind Nebenreaktionen nicht auszuschließen; beispielsweise setzt die in Gegenwart basischer Katalysatoren auftretende Trimerisierung des Methylisocyanats die Ausbeute an gewünschtem Harnstoff stark herab. Ferner ist bekannt (Chem. Ber. 115 (1982), S. 919; Chem. Ber. 117, (1984), S. 1707), daß die Umsetzung der wenig reaktiven Diarylamine mit Isocyanaten gelingt, die eine stark elektronenanziehende Gruppe aufweisen, beispielsweise mit $(CF_3CO)NCO$ oder $SF_5$-NCO. Solche Isocyanate sind technisch schwer handhabbar und die genannten elektronenanziehenden Gruppen sind anschließend nicht gegen andere gewünschte Substituenten austauschbar.

Es wurde nun ein Verfahren zur Herstellung von N,N-Diarylharnstoffen der Formel

$R^1$-$NR^2$-CO-NH-$R^3$,

in der
$R^1$ und $R^2$ Aryl bedeuten und
$R^3$ Alkyl, Aralkyl oder Aryl bedeutet,
gefunden, das dadurch gekennzeichnet ist, daß man Diarylamine der Formel

$R^1$-NH-$R^2$

mit Isocyanaten der Formel

$R^3$-NCO,

worin
$R^1$, $R^2$ und $R^3$ die obige Bedeutung haben,
in Gegenwart sauer reagierender Verbindungen und gegebenenfalls in Gegenwart eines inerten Lösungs- und/oder Verdünnungsmittels bei erhöhter Temperatur umsetzt.

Aryl ist einkerniges oder mehrkerniges Aryl, wie Phenyl, Naphthyl oder Anthryl, bevorzugt Phenyl oder Naphthyl. Das genannte Aryl kann unsubstituiert oder substituiert sein; als Substituenten kommen 1 bis 3, bevorzugt 1 oder 2 Substituenten, besonders bevorzugt 1 Substituent aus der Gruppe der niederen Alkylgruppen mit 1 bis 4 C-Atomen (Methyl, Ethyl, Propyl, Isopropyl, Butyl, Isobutyl), der niederen Alkoxygruppen mit 1 bis 4 C-Atomen (Methoxy, Ethoxy, Propoxy, Isopropoxy, Butoxy, Isobutoxy) oder der Halogene (Fluor, Chlor, Brom) in Frage. Weitere Substituenten können sein: die Nitrogruppe, die Nitrosylgruppe sowie die Acetylgruppe.

Alkyl ist ein geradkettiger oder verzweigter gesättigter Kohlenwasserstoffrest mit 1-12 C-Atomen, bevorzugt 1-8 C-Atomen, besonders bevorzugt 1-4 C-Atomen, wie Methyl, Ethyl, Propyl, Isopropyl, Butyl, Isobutyl, Hexyl, Octyl oder Dodecyl. Solche Kohlenwasserstoffreste können weiterhin ein- oder mehrfach durch Halogen (Fluor, Chlor oder Brom) substituiert sein. Solche Halogenalkylreste sind beispielsweise Trifluormethyl, Trichlormethyl, Tribrommethyl, ganz oder teilweise fluoriertes, chloriertes oder bromiertes Ethyl, Propyl, Butyl, Hexyl, Octyl oder Dodecyl.

Als Aralkyl sei beispielsweise Benzyl, Phenylethyl, Phenyl-propyl, Naphthyl(1- oder 2-)-methyl, Naphthyl-ethyl oder deren Homologe genannt. Die aromatischen Kerne in den Aralkylgruppen können die gleichen Substituenten tragen, die oben für Aryl angeführt sind.

Bevorzugte Diarylamine für das erfindungsgemäße Verfahren sind solche der Formel

$R^{11}$-NH-$R^{12}$,

worin
$R^{11}$ und $R^{12}$ unabhängig voneinander sind und Phenyl, Naphthyl, $C_1$ bis $C_4$-Alkylphenyl oder $C_1$ bis $C_4$-Alkylnaphthyl bedeuten.

Besonders bevorzugte Diarylamine für das erfindungsgemäße Verfahren sind solche der Formel

$R^{21}$-NH-$R^{22}$

worin
$R^{21}$ und $R^{22}$ Phenyl bedeuten.

Beispiele für erfindungsgemäß einsetzbare Diarylamine sind: Diphenylamin, Phenyl-naphthylamin und Ditolylamin, bevorzugt Diphenylamin.

Bevorzugte Isocyanate für das erfindungsgemäße Verfahren sind solche der Formel

$R^{13}$-NCO,

worin
$R^{13}$ $C_1$ bis $C_8$-Alkyl, Phenyl, Naphthyl, $C_1$ bis $C_4$-Alkylphenyl sowie Halogenphenyl bedeutet.

Besonders bevorzugte Isocyanate für das erfindungsgemäße Verfahren sind solche der Formel

$R^{23}$-NCO

worin
$R^{23}$ Methyl, Ethyl, Propyl, Butyl, Isobutyl, Phenyl, Chlorphenyl oder Tolyl bedeutet.

Beispiele für erfindungsgemäß einsetzbare Isocyanate sind: Methylisocyanat, Butylisocyanat, Phenylisocyanat, bevorzugt Methylisocyanat.

Das erfindungsgemäße Verfahren wird in Gegenwart einer oder mehrerer sauer reagierender Verbindungen durchgeführt. Als sauer reagierende

Verbindungen werden in das erfindungsgemäße Verfahren solche eingesetzt, die dem sogenannten Brönsted-Typ (also eine Verbindung, die ein Proton abspalten kann) zuzurechnen sind. Die sauer reagierenden Verbindungen können anorganischer oder organischer Natur sein. Als sauer reagierende Verbindungen für das erfindungsgemäße Verfahren kommen demnach Verbindungsklassen wie die folgenden in Betracht:

a) anorganische Säuren, wie Phosphorsäure;
b) teilweise veresterte mehrbasische Säuren, die also noch eine Säurefunktion enthalten, wie Monoalkylester der Schwefelsäure, Mono- oder Dialkylester der Phosphorsäure, wobei die Alkylgruppe 1 bis 8 C-Atome, bevorzugt 1 bis 4 C-Atome enthält;
c) aliphatische, aromatische oder heterocyclische Mono- oder Polycarbonsäuren;
d) Teilester von organischen Polycarbonsäuren;
e) aliphatische oder aromatische Sulfonsäuren oder Phosphonsäuren;
f) sauer reagierende Ionenaustauscherharze.

Als Beispiele für solche erfindungsgemäß einsetzbaren sauer reagierenden Verbindungen seien genannt: $H_3PO_4$, n-Butylphosphat, Di-n-butylphosphat, Essigsäure, Propionsäure, Benzoesäure, Methansulfonsäure, p-Toluolsulfonsäure und andere.

Bevorzugt werden in das erfindungsgemäße Verfahren eingesetzt: Di-n-butylphosphat, Essigsäure, Benzoesäure und p-Toluolsulfonsäure.

Die genannten sauer reagierenden Verbindungen können nach der Abtrennung der Reaktionsprodukte, gegebenenfalls im Gemisch mit nicht umgesetzten Ausgangsstoffen und gegebenenfalls im Gemisch mit Lösungs- oder Verdünnungsmittel in einen weiteren Reaktionsansatz des erfindungsgemäßen Verfahrens zurückgeführt werden. Diese Rückführung kann auch mehrfach erfolgen.

Als Lösungs- bzw. Verdünnungsmittel kommen inerte Flüssigkeiten in Betracht, die mit den Reaktionsteilnehmern keine Reaktion eingehen, beispielsweise aliphatische oder aromatische Kohlenwasserstoffe oder aliphatische oder aromatische Halogen-Kohlenwasserstoffe.

Beispiele für geeignete Lösungs- und/oder Verdünnungsmittel sind: Toluol, Xylol, Diethylether sowie Chloroform.

Für das erfindungsgemäße Verfahren werden 0,8 bis 2 Mol, bevorzugt 0,9 bis 1,5 Mol, besonders bevorzugt 1.0 bis 1,2 Mol Isocyanat pro Mol Diarylamin eingesetzt.

Für das erfindungsgemäße Verfahren werden 0,001 bis 0,3 Mol, bevorzugt 0,002 bis 0,1 Mol, besonders bevorzugt 0,003 bis 0,08 Mol der sauer reagierenden Verbindung pro Mol Diarylamin eingesetzt.

Das Lösungs- oder Verdünnungsmittel oder ein Gemisch mehrerer von ihnen wird in einer Menge von 50 bis 2000, bevorzugt 100 bis 1000, besonders bevorzugt 200 bis 600 Milliliter pro Mol Diarylamin eingesetzt.

Das erfindungsgemäße Verfahren wird bei erhöhter Temperatur, beispielsweise bei 40 bis 160°C, bevorzugt 80 bis 140°C, besonders bevorzugt 90 bis 130°C durchgeführt.

Das erfindungsgemäße Verfahren, insbesondere die Reihenfolge der Zugabe der Reaktionspartner, kann nach verschiedenen Varianten durchgeführt werden. So kann beispielsweise das Diarylamin zusammen mit dem Isocyanat und der sauer reagierenden Verbindung in einem inerten Lösungs- bzw. Verdünnungsmittel vorgelegt werden, worauf der Reaktionsansatz unter Rühren auf die gewählte Reaktionstemperatur gebracht wird. Sollte die gewählte Reaktionstemperatur oberhalb der des Siedepunktes des Lösungs- bzw. Verdünnungsmittels liegen, kann in einer dem Fachmann bekannten Art und Weise bei erhöhtem Druck gearbeitet werden. Ansonsten ist das erfindungsgemäße Verfahren vom Druck unabhängig. In vielen Fällen fällt der Harnstoff während der Reaktion oder nach dem Abkühlen auf Raumtemperatur aus und kann so auf einfache Weise isoliert werden. Die Zugabe der sauer reagierenden Verbindung kann außer der beschriebenen Art jedoch auch so durchgeführt werden, daß sie mit einer der Reaktionskomponenten zugefügt wird oder teilweise vorgelegt und teilweise zugefügt wird. So kann es vorteilhaft sein, die sauer reagierende Verbindung mit einer Reaktionskomponente und dem Lösungs- bzw. Verdünnungsmittel vorzulegen und die andere Reaktionskomponente portionsweise zuzugeben. Weiterhin kann eine Reaktionskomponente im Lösungs- bzw. Verdünnungsmittel vorgelegt werden und die sauer reagierende Verbindung zusammen mit der anderen Reaktionskomponente zudosiert werden. Dieses Zudosieren kann vor, während oder nach dem Aufheizen auf die Reaktionstemperatur erfolgen.

Weiterhin ist es grundsätzlich möglich, bei Reaktionsgemischen die auch ohne ein Lösungs- bzw. Verdünnungsmittel flüssig sind, auf dieses Lösungs- bzw. Verdünnungsmittel zu verzichten. Es ist jedoch bevorzugt, ein Lösungs- bzw. Verdünnungsmittel im Rahmen der oben angegebenen Mengen zu verwenden.

Erfindungsgemäß ist der Zusatz von sauer reagierenden Verbindungen ohne Beeinträchtigung des eingesetzten Diarylamins oder des eingesetzten Isocyanats möglich. Dies erlaubt eine erhebliche Verkürzung der Reaktionszeiten und Herabsetzung der Reaktionstemperaturen bei der Umsetzung der ansonsten nur träge reagierenden Diarylamine mit Isocyanaten. Unter diesen milderen Bedingungen werden deutlich bessere Raum-Zeit-Ausbeuten erzielt.

Es ist besonders überraschend, daß mit dem erfindungsgemäßen Verfahren solch gute Raum-Zeit-Ausbeuten an N,N-Diarylharnstoffen erzielt werden, da es beispielsweise aus Houben-Weyl, Methoden der organischen Chemie, Band VIII, Seite 129 (1952) sowie aus Becker, Braun; Kunststoff-Handbuch, 7 Polyurethane, Carl Hanser Verlag, München/Wien, Seite 8 (1983) bekannt ist, daß Verbindungen mit sauren Eigenschaften, z.B. Chlorwasserstoff, Acetylchlorid, Phosphorpentachlorid sowie andere Mineralsäuren, bei der Umsetzung von Isocyanaten mit Verbindungen mit aktiven Wasserstoffatomen, wie Amine, reaktionsverzögernd

wirken. Es mußte also erwartet werden, daß die erfindungsgemäß eingesetzten sauer reagierenden Verbindungen die Umsetzung verzögern würden und so die Raum-Zeit-Ausbeute verminderten.

Die erfindungsgemäß herstellbaren N,N-Diarylharnstoffe finden als Stabilisatoren (z.B. Akardit II®) oder als Herbizide im Pflanzenschutz Anwendung.

Beispiele 1 bis 5

0,5 Mol Diphenylamin wurden in 150 ml Toluol vorgelegt. Dazu gab man 0,53 Mol Methylisocyanat und 1 g sauer reagierender Verbindung (siehe Tabelle 1). Man erwärmte 8 Stunden unter Rühren auf 100°C, ließ auf Raumtemperatur abkühlen und filtrierte den gebildeten N-Methyl-N',N'-diphenylharnstoff ab. Der Harnstoff wurde mit 100 ml kaltem Toluol gewaschen. Ausbeute siehe Tabelle 1.

Tabelle 1

Abhängigkeit der Ausbeute an N-Methyl-N' ,N'-diphenylharnstoff vom verwendeten Katalysator

| Beispiel | Katalysator | Ausbeute (bez. auf Diphenylamin) |
|---|---|---|
| 1 | Di-n-butylphosphat | 95% |
| 2 | Essigsäure | 90% |
| 3 | Benzoesäure | 95% |
| 4 | Methansulfonsäure | 94% |
| 5 | p-Toluolsulfonsäure | 95% |

Beispiel 6 (Vergleichsbeispiel)

Die unter Beispiel 1 beschriebene Umsetzung zu N-Methyl-N' ,N'-diphenylharnstoff erforderte ohne Zusatz an sauer reagierender Verbindung bei sonst gleichen Bedingungen eine Reaktionszeit von 3 Tagen um eine Ausbeute von 85 % (bezogen auf Diphenylamin) zu erlangen.

Beispiel 7

0,5 Mol α-Naphthyl-phenylamin wurden zusammen mit 0,53 Mol Methylisocyanat und 0,6 g Di-n-butylphosphat in 150 ml Toluol vorgelegt. Man erwärmte 24 Stunden unter Rühren auf 100° C und kühlte dann auf 10° C ab. Nach dem Abfiltrieren und Waschen mit 150 ml kaltem Toluol erhielt man nach dem Trocknen 0,4 Mol N-Methyl-N'-phenyl-N'-α-naphthyl-harnstoff. Fp.: 146°C/Ausbeute: 80 % d.Th.

Beispiel 8

0,5 Mol Diphenylamin wurden zusammen mit 0,53 Mol Phenylisocyanat und 0,6 g Di-n-butylphosphat in 150 ml Toluol vorgelegt. Man erwärmte 24 Stunden unter Rühren auf 100°C und kühlte dann auf Raumtemperatur ab. Bei ca. 30°C begann der Harnstoff auszukristallisieren. Nach dem Abfiltrieren und Waschen mit 150 ml kaltem Toluol erhielt man nach dem Trocknen 0,41 Mol N-Phenyl-N' ,N'-diphenylharnstoff. Fp.: 131° C/Ausbeute: 82 % d.Th.

Beispiel 9

0,5 Mol Diphenylamin wurden zusammen mit 0,53 Mol n-Butylisocyanat und 1 g Di-n-butylphosphat in 150 ml Xylol vorgelegt. Man erwärmte unter Rühren 24 Stunden auf 120° C und kühlte dann auf Raumtemperatur ab. Nach dem Abfiltrieren und Waschen mit 100 ml kaltem Xylol erhielt man nach dem Trocknen 0,3 Mol N-Butyl-N' ,N'-Diphenylharnstoff. Fp.: 92° C/Ausbeute: 60 % d.Th.

Beispiel 10

0,5 Mol Diphenylamin wurden zusammen mit 0,53 Mol Methylisocyanat und 0,6 g Di-n-butylphosphat in 150 ml Toluol vorgelegt. Man erwärmte unter Rühren 10 Stunden auf 100°C. Nach dem Abkühlen auf Raumtemperatur saugte man den gebildeten N-Methyl-N' ,N'-diphenylharnstoff ab. Das Filtrat wurde mit Toluol auf 150 ml ergänzt und mit 0,5 Mol Diphenylamin und 0,53 Mol Methylisocyanat versetzt. Man erwärmte wiederum 10 Stunden unter Rühren auf 100° C und kühlte dann auf Raumtemperatur ab. Die Rückführung des Filtrates und die darauffolgende Zugabe von jeweils 0,5 Mol Diphenylamin und 0,53 Mol Methylisocyanat wurde noch dreimal wiederholt. Man erhielt nach diesen fünf Umsetzungen bei nur einmaliger Zugabe des Katalysators 2,4 Mol N-Methyl' ,N'-diphenylharnstoff. Der Harnstoff wurde nach jeder Umsetzung mit jeweils 100 ml kaltem Toluol gewaschen. Fp.: 170° C/Ausbeute: 96 % d.Th.

**Patentansprüche**

1. Verfahren zur Herstellung von N,N-Diarylharnstoffen der Formel
$R^1$-$NR^2$-CO-NH-$R^3$,
in der
$R^1$ und $R^2$ Aryl bedeuten und
$R^3$ Alkyl, Aralkyl oder Aryl bedeutet,
dadurch gekennzeichnet, daß man Diarylamine der Formel
$R^1$-NH-$R^2$
mit Isocyanaten der Formel
$R^3$-NCO,
worin
$R^1$, $R^2$ und $R^3$ die obige Bedeutung haben,
in Gegenwart sauer reagierender Verbindungen und gegebenenfalls in Gegenwart eines inerten Lösungs-und/oder Verdünnungsmittels bei erhöhter Temperatur umsetzt.

2. Verfahren nach Anspruch 1, dadurch gekennzeichnet, daß Diarylamine der Formel
$R^{11}$-NH-$R^{12}$,
eingesetzt werden, worin
$R^{11}$ und $R^{12}$ unabhängig voneinander sind und Phenyl, Naphthyl, $C_1$ bis $C_4$-Alkylphenyl oder $C_1$ bis $C_4$-Alkylnaphthyl bedeuten.

3. Verfahren nach Ansprüchen 1 und 2, dadurch gekennzeichnet, daß Diarylamine der Formel
$R^{21}$-NH-$R^{22}$,
eingesetzt werden, worin
$R^{21}$ und $R^{22}$ Phenyl bedeuten.

4. Verfahren nach Ansprüchen 1 bis 3, dadurch gekennzeichnet, daß Isocyanate der Formel
$R^{13}$-NCO
eingesetzt werden, worin
$R^{13}$ $C_1$ bis $C_8$-Alkyl, Phenyl, Naphthyl, $C_1$ bis $C_4$-Phenyl oder Halogenphenyl bedeutet.

5. Verfahren nach Ansprüchen 1 bis 4, dadurch gekennzeichnet, daß Isocyanate der Formel
$R^{23}$-NCO,
eingesetzt werden, worin
$R^{23}$ Methyl, Ethyl, Propyl, Butyl, Isobutyl, Phenyl, Chlorphenyl oder Tolyl bedeutet.

6. Verfahren nach Ansprüchen 1 bis 5, dadurch gekennzeichnet, daß 0,8 bis 2 Mol, bevorzugt 0,9 bis 1,5 Mol, besonders bevorzugt 1,0 bis 1,2 Mol Isocyanat pro Mol Diarylamin eingesetzt werden.

7. Verfahren nach Ansprüchen 1 bis 6, dadurch gekennzeichnet, daß 0,001 bis 0,3 Mol, bevorzugt 0,002 bis 0,1 Mol, besonders bevorzugt 0,003 bis 0,08 Mol sauer reagierende Verbindung pro Mol Diarylamin eingesetzt werden.

8. Verfahren nach Ansprüchen 1 bis 7, dadurch gekennzeichnet, daß als sauer reagierende Verbindung anorganische und organische Brönstedt-Säuren eingesetzt werden.

## Claims

1. Process for the preparation of N,N-diaryl-ureas of the formula
$R^1$–N$R^2$–CO–NH–$R^3$
in which
$R^1$ and $R^2$ denote aryl and
$R^3$ denotes alkyl, aralkyl or aryl,
characterized in that diarylamines of the formula
$R^1$–NH–$R^2$
are reacted with isocyanates of the formula
$R^3$–NCO
wherein
$R^1$, $R^2$ and $R^3$ have the above meaning, in the presence of acid compounds and if appropriate in the presence of an inert solvent and/or diluent at elevated temperature.

2. Process according to Claim 1, characterized in that diarylamines of the formula
$R^{11}$–NH–$R^{12}$
wherein
$R^{11}$ and $R^{12}$ are independent of one another and denote phenyl, naphthyl, $C^1$ to $C^4$-alkylphenyl or $C_1$ to $C_4$-alkylnaphthyl, are employed.

3. Process according to Claims 1 and 2, characterized in that diarylamines of the formula
$R^{21}$–NH–$R^{22}$
wherein $R^{21}$ and $R^{22}$ denote phenyl, are employed.

4. Process according to Claims 1 to 3, characterized in that isocyanates of the formula
$R^{13}$–NCO
wherein
$R^{13}$ denotes $C_1$ to $C_8$-alkyl, phenyl, naphthyl, $C_1$ to $C_4$-phenyl or halogenophenyl, are employed.

5. Process according to Claims 1 to 4, characterized in that isocyanates of the formula
$R^{23}$–NCO
wherein
$R^{23}$ denotes methyl, ethyl, propyl, butyl, isobutyl, phenyl, chlorophenyl or tolyl, are employed.

6. Process according to Claims 1 to 5, characterized in that 0.8 to 2 mol, preferably 0.9 to 1.5 mol and particularly preferably 1.0 to 1.2 mol of isocyanate are employed per mol of diarylamine.

7. Process according to Claims 1 to 6, characterized in that 0.001 to 0.3 mol, preferably 0.002 to 0.1 mol and particularly preferably 0.003 to 0.08 mol of acid compound are employed per mol of diarylamine.

8. Process according to Claims 1 to 7, characterized in that inorganic and organic Brönstedt acids are employed as the acid compound.

## Revendications

1. Procédé pour la fabrication de N,N-diarylurée de formule
$R^1$–N$R^2$–CO–NH–$R^3$,
dans laquelle
$R^1$ et $R^2$ représentent chacun un reste aryle et
$R^3$ représente un reste alkyle, arylalkyle ou aryle,
caractérisé en ce que l'on fait réagir des diarylamines de formule
$R^1$–NH–$R^2$
avec des isocyanates de formule
$R^3$–NCO,
dans lesquelles
$R^1$, $R^2$ et $R^3$ ont la signification ci-dessus, à température élevée en présence de composés à réaction acide et éventuellement en présence d'un solvant et/ou diluant inerte.

2. Procédé selon la revendication 1, caractérisé en ce que l'on utilise des diarylamines de formule
$R^{11}$–NH–$R^{12}$,
dans laquelle
$R^{11}$ et $R^{12}$ représentent chacun, indépendamment l'un de l'autre, un reste phényle, naphtyle, (alkyl en $C_1$–$C_4$)phényle ou (alkyl en $C_1$–$C_4$)naphtyle.

3. Procédé selon les revendications 1 et 2, caractérisé en ce qu'on utilise des diarylamines de formule
$R^{21}$–NH–$R^{22}$,
dans lesquelles
$R^{21}$ et $R^{22}$ sont des restes phényles.

4. Procédé selon les revendications 1 à 3, caractérisé en ce que l'on utilise des isocyanates de formule
$R^{13}$–NCO,
dans laquelle
$R^{13}$ représente un reste alkyle en $C_1$–$C_8$, phényle, naphtyle, (alkyl en $C_1$–$C_4$)phényle ou halogénophényle.

5. Procédé selon les revendications 1 à 4, caractérisé en ce que l'on utilise des isocyanates de formule
$R^{23}$–NCO,
dans laquelle
$R^{23}$ représente un reste méthyle, éthyle, propyle, butyle, iso-butyle, phényle, chlorophényle ou tolyle.

6. Procédé selon les revendications 1 à 5, caractérisé en ce que l'on utilise 0,8 à 2 mol d'isocyanate,

de préférence 0,9 à 1,5 mol, en particulier 1,0 à 1,2 mol d'isocyanate par mole de diarylamine.

7. Procédé selon les revendications 1 à 6, caractérisé en ce que l'on utilise 0,001 à 0,3 mol, de préférence 0,002 à 0,1 mol, en particulier 0,003 à 0,08 mol de composé à réaction acide par mole de diarylamine.

8. Procédé selon les revendications 1 à 7, caractérisé en ce que l'on utilise comme composés à réaction acide des acides de Brönstedt inorganiques et organiques.